# EUROPEAN PATENT SPECIFICATION

(11) **EP 2 999 394 B1**
(45) Date of publication and mention of the grant of the patent: **09.08.2017**
(21) Application number: 14726734.8
(22) Date of filing: 21.05.2014
(51) Int. Cl.: A61B 5/00, A61B 5/103, A61B 5/11, A47C 31/12

(54) **POSTURE MONITOR**
HALTUNGSMONITOR
MONITEUR DE POSITION

(30) Priority: 21.05.2013 GB 201309169
(43) Date of publication of application: 30.03.2016
(73) Proprietor: Sutton, Richard, Cheltenham Gloucestershire GL52 8TH (GB)
(72) Inventor: Sutton, Richard, Cheltenham Gloucestershire GL52 8TH (GB)
(74) Representative: Games, Robert Harland
(86) International application number: PCT/GB2014/051556
(87) International publication number: WO 2014/188187

(56) References cited:
- WO-A1-2008/119106
- CN-U- 201 920 164
- US-A1- 2011 275 939
- US-A1- 2012 245 491
- US-B1- 6 648 838

## Description

The present invention relates to a posture monitor and particularly to a posture monitor for monitoring the posture of a seated person to improve their posture and mobility whilst seated.

### BACKGROUND TO THE INVENTION

Good posture and mobility is essential for maintaining a healthy back and in particular for preventing back pain and other problems from developing. People who spend significant periods of time sat down in the same position, for example, office workers, are known to be particularly susceptible to back ache or injury due to poor posture and lack of movement. A person is likely to slouch in a chair after being sat down for some time, and this, combined with a lack of movement, can result in back pain.

In the UK, a business is obliged to protect its employees from injury in the workplace. Helping office workers to correct their posture and to move their joints and limbs at regular intervals can improve both health and productivity. Statistics suggest that up to 80% of workers in the UK take time off, attributed to back pain. Dehydration in the workplace is also a significant cause of headaches at work.

Good posture whilst seated is simple to achieve, but can be difficult for a person to maintain, especially where their minds are distracted by other matters. Various devices have therefore been proposed which monitor a person's posture, and provide a simple reminder if correct posture is not maintained. These devices typically include some form of pressure transducer, which may be mounted to a seat back. For example, CN201920164U discloses a "posture correcting chair". However, such devices are not able to be used with any chair, and this is a serious disadvantage. Many people have a strong preference regarding the type of chair which they will sit on, and will be unlikely to use a posture correcting chair where a more comfortable chair which is a preferred shape is available.

Other known devices do not mount to a chair, but are worn on the body. However, these devices may well be uncomfortable, and in practice will have to be worn all day, even though posture correction may only be required when seated.
A further problem with known devices is that they are often unable to distinguish between a situation where no person is seated on a chair, and where a person is seated on the chair but is not making contact with the seat back, possibly because they have their back arched forwards, hunched over a computer keyboard or document. Either the device will not alert to this potentially very poor posture, or the device will alert incorrectly when no person is sat in the chair, drawing unnecessary power and possibly depleting a battery, and causing annoyance to people in the surrounding area. Document US 2012/0245491 A1 discloses a posture monitor according to the preamble of claim 1.

It is an object of this invention to reduce or obviate the above mentioned problems.

### STATEMENT OF INVENTION

According to the present invention, there is provided a posture monitoring device according to claim 1, for use with a seat, comprising at least two pressure sensors, a mount for securing the pressure sensors to a seat back, the mount including a hook for fitting over the seat back and a flexible strap depending from the hook, the sensors being spaced apart and mounted to the strap, and output means for alerting when the pressure measured on at least one of the pressure sensors falls outside of a predetermined range.
The hook may be a malleable hook. The sensors may be adjustably or movably mounted to the strap.
The malleable hook may be formed into a shape to fit a particular seat back by manual bending. Advantageously, the device may be fitted to substantially any seat. The pressure sensors are movable with respect to the seat back, and as such they may be repositioned to suit different types of seat. For example, the pressure sensors may be moved further away from the hook for use with a high-backed chair. The movable pressure sensors also provide for adjustability to suit different users.

Typically, the device may comprise two pressure sensors, disposed substantially one above the other on the mount. Pressure on both the pressure sensors indicates that the user is sat on the seat, with his back substantially upright against the seat back in a good postural position. Pressure on only one of the pressure sensors may indicate that the user is slouched in the seat in a poor postural position.

The output means may further indicate when the pressure on both of the pressure sensors falls outside of a predetermined range. This may indicate that the user is sat with his back away from the seat back, possibly hunched over a desk.

The output means may include a first lamp which illuminates when the pressure on both of the pressure sensors is within a predetermined range, a second lamp which illuminates when the pressure on at least one of the pressure sensors is outside of the predetermined range, and a third lamp which illuminates when the pressure on both of the pressure sensors is outside of the predetermined range. In this way, a "traffic light" system provides the user with instant and easy to understand feedback on his posture. The first lamp may be green, indicating correct posture. The second lamp may be yellow, indicating less-than-perfect posture. The third lamp may be red, indicating poor posture.

An audible alert may be provided when the pressure on both of the pressure sensors falls outside of a predetermined range. This will prompt the user to correct his poor posture. The audible alert may be a bell, buzzer, or the like, or may be a recorded or synthesized voice alert.

A vibrating alert may additionally or alternatively be provided when the pressure on one or both of the pressure sensors falls outside of a predetermined range. A vibrating alert is advantageous, because it can be felt by the user, but does not disturb others in the vicinity.

The device may automatically switch off after a predetermined time period during which no pressure is detected by either pad. Where the device is powered by batteries, the charge may be quickly depleted where the device continues to alert when, in fact, no person is sat on the chair. A time-delay switch off prevents this.

A light sensor may be provided, the device automatically switching off when the light level detected by the sensor is greater than a predetermined threshold. A light sensor provides a useful means for detecting whether a person is sat in a seat, even when their back is not in contact with the pressure sensors. Typically, a person sat on a seat will substantially obscure the seat back from ambient light, even if they are sat reasonably far forward on the seat, away from the seat back.

A light sensor will, however, not work reliably in a dark room. It is therefore particularly advantageous to provide both a light sensor and a time delay shut-off. Where the light sensor detects that the seat is unoccupied, the system may immediately shut down. Where the pressure sensors both detect no pressure, an alert may be raised, indicating poor posture. If the lack of pressure continues for more than a predetermined period of time, then the system may eventually shut down.

The lamps may be provided on a disconnectable and repositionable remote output unit or module. This allows the lamps to be placed in view of the user, for example on a desk. The remote output unit may be connected to the rest of the device by means of a cable, in which case it may be disconnectable by means of a plug-and-socket connection. Alternatively, the remote output unit may communicate with the other parts of the device by wireless means. Where the lamps are not required (e.g. where an audible or vibrating alert is deemed sufficient, and where such alert is provided outside of the remote output unit) the remote output unit may be disconnected, and the rest of the device may continue to function normally.

The vibrating alert may be provided by a transducer mounted in a housing at a base of the seat back. This provides a convenient arrangement, since the pressure pads can be mounted on a substantially vertical member extending downwards from the hook, which secures the vertical member to the top of the seat back. Cabling or other connection means may then pass along the substantially vertical member, to connect with the vibrating transducer in the housing at the base of the seat back. A connector for attaching the remote output unit, an audio transducer, controls or controller, and/or an auxiliary output connector may also be provided in the same housing.

The audible alert may be provided by a transducer mounted within the disconnectable and repositionable remote output unit. The audible alert may alternatively be provided by a transducer mounted within the housing at the base of the seat back.

A display screen, for example a liquid crystal display, may be provided. The display screen may be mounted in the remote output unit. The remote output unit may be provided as a wand, which may be attached to the top of a computer screen or rested on a desk. The wand may incorporate a webcam.

The LCD display may provide more detailed feedback. For example, it may display suggestions for particular exercises to improve posture, such as a series of neck rolls, for example.

Auxiliary output means may be provided for the connection of a recording, monitoring, or output device. In this way, different types of visual, audio, or vibrating output means may be attached to the device to suit the needs of the user. Also, the device may be connected to, for example, a personal computer, to allow the user's sitting habits to be recorded or monitored over a period of time. This also allows for alerts to be displayed on the computer display. Software may also be provided to interrupt the normal operation of the computer, providing the user with an incentive to correct his posture.

Where auxiliary output means are provided, exercise options for improving posture or generally ensuring good health may be presented to the user in the form of automatic links to websites and/or online videos. In use, poor posture may be detected.

Conveniently, the auxiliary output means is provided by a USB connector. This allows for attachment to all modern computers, and many other devices.

The device may incorporate a timer, and alert means may be activated when a user has been sat on the chair (whether or not with good posture) for more than a predetermined period of time. The alert means may be accompanied by suggestions of exercises via display screens and/or a personal computer attached to auxiliary output means.

By providing a timer, alert means, and exercise suggestions, a user is reminded not to spend too long sat on his chair, and to take appropriate exercise breaks. For example, at 40 minute intervals, or other intervals to one's preference, the wand may display animation of simple mobility exercises to help mobilise limbs and joints. A reminder to "take a drink" may also be displayed. Good hydration can prevent headaches in the workplace.
Conveniently the remote output unit, or wand, may be hard wired or wirelessly connected to a computer, facilitating the downloading of selected exercises displayed on the computer screen in a web browser displaying an internet site. The wand or controller may store the exercises, and they can be overwritten by new downloaded exercises, as desired. The wand may incorporate a button mounted on the housing of the wand for initiating connection to the desired website on the browser. The wand may also be adapted to connect with a mobile telephone, tablet or other smart device for enabling images of mobility exercise routines to be transferred to the wand. The repetitive display throughout the day of mobility exercises and stretches promotes good mobility and helps to prevent people becoming "cast" in their chair.

According to a second aspect of the present disclosure, there is provided a posture monitoring device for use with a seat, comprising at least two pressure sensors, a mount for movably securing the pressure sensors to a seat back, the mount including a malleable hook for fitting over the seat back, and output means for alerting when the pressure measured on at least one of the pressure sensors falls outside of a predetermined range.

### DESCRIPTION OF THE DRAWINGS

For a better understanding of the present invention, and to show more clearly how it may be carried into effect, reference will now be made by way of example only to the accompanying drawings, in which:
Figure 1 shows a perspective view of a posture monitoring device;
Figure 2 shows a perspective view of the posture monitoring device of Figure 1, mounted to a chair;
Figure 3 shows an alternative perspective view of the posture monitoring device and chair of Figure 2; and
Figure 4 shows a schematic perspective view of an alternative embodiment of the output means disposed on top of a computer screen.

### DESCRIPTION OF PREFERRED EMBODIMENT

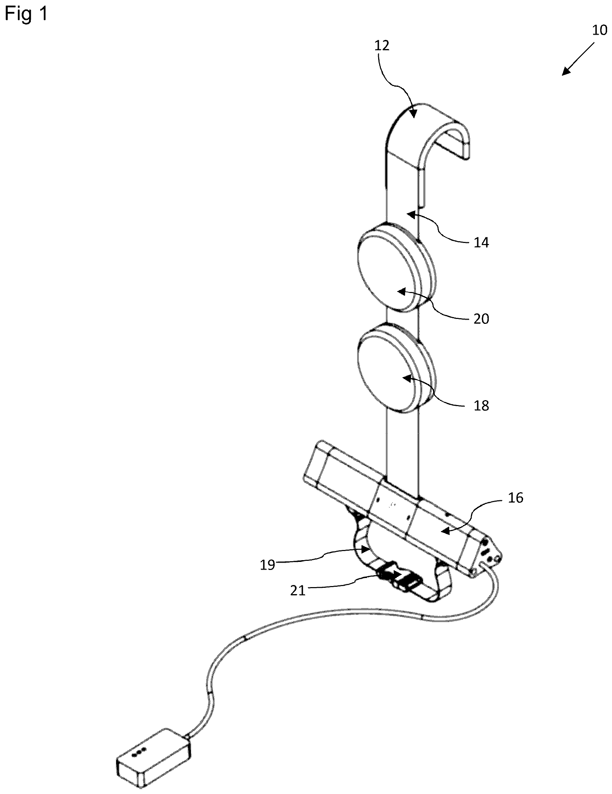

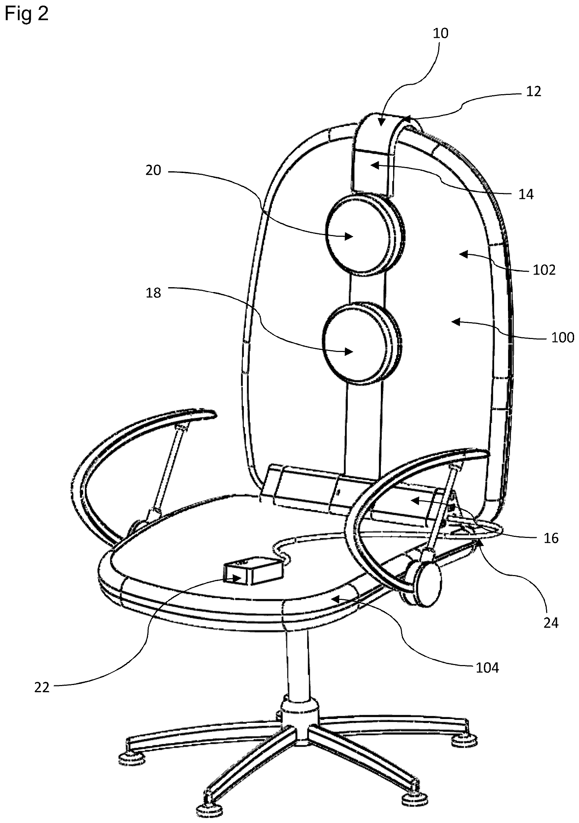

Referring to Figures 1 to 3, a posture monitoring device is indicated generally at 10. The posture monitoring device 10 includes a malleable hook 12, and a flexible strap 14 extending downwardly from the hook 12. The flexible strap 14 is attached at one end to the malleable hook 12, and at the other end to a housing 16. A control unit is disposed within the housing. Two pressure sensors 18, 20 are mounted to the strap, spaced apart along the length of the strap 14 between the hook 12 and the housing 16. The spacing of the pressure sensors 18, 20 on the strap is adjustable.

The malleable hook 12 is plastically deformable, so that is can be manually deformed into various shapes for attachment to different chairs. The hook 12 may comprise an inner metallic portion and an outer plastics portion.

The strap 14 may be made from, for example, nylon. The strap 14 is flexible and so can conform to the shape of various different chair backs. Adjustment means are provided for adjusting the length of the strap between the hook 12 and the housing 16, to suit chairs with backs of different heights.

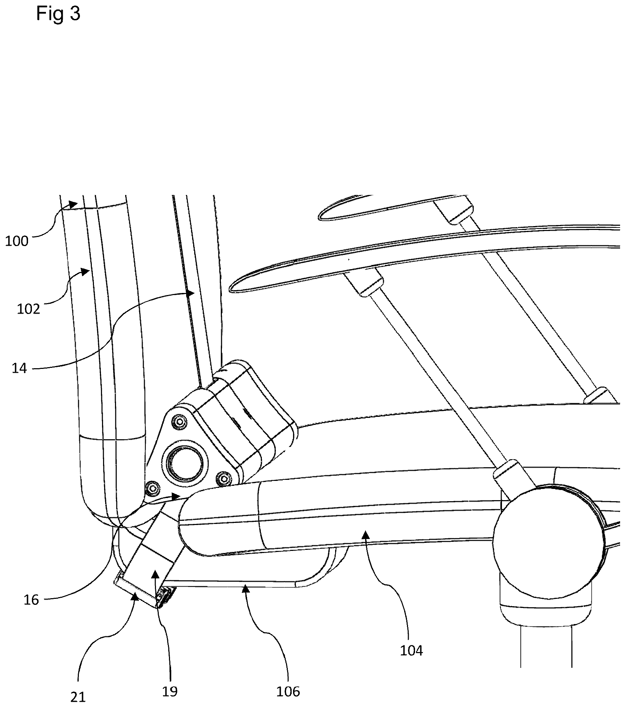

The housing 16 is in the shape of an elongate triangular prism. This shape is useful because it allows the housing 16 to fit at the rear of a chair, between the seat and the back of the chair, as shown best in Figure 3. In this position, the housing 16 does not obstruct a person sitting on the chair. The strap 14 attaches to the housing 16 at an apex of the triangular housing, mid-way along its length, and is retractable into a spring loaded cassette within the housing for adjusting the length of the strap 14.

An attachment strap 19 is provided on the base of the housing 16, that is, on a face of the housing opposite the apex to which strap 16 is attached. The attachment strap 19 is provided with releasable fastening means 21. In this embodiment, the releasable fastening means 21 is a plastic buckle-clip. The releasable fastening means 21 includes length adjustment means for adjusting the length of the attachment strap 19.

The pressure sensors 18, 20 are movable on the strap, and are connected to the housing 16 via wires. The wires may be at least in part interwoven into the fabric of the strap 14. The wires may be substantially entirely woven into the fabric.

In use, as seen in Figure 3, the housing may be strapped to the base of the seat. In the Figure, the chair 100 is of the type with a separate back 102 and seat 104, and a narrow connecting member 106 between the back and the seat. This type of construction is typical of an office chair. It will be appreciated that the attachment strap 19 may be lengthened to fit to a chair with a solid connection all the way along the width of the back and the seat, as is typical of, for example, dining chairs.

To install the device on a chair 100, the malleable hook 12 is first deformed to fit around the top of the chair back 102. The housing 16 is then strapped to the base of the chair 100 with the attachment strap 19, between the seat 104 and the back 102, and the length of the strap 14 is adjusted so that it extends tightly between the hook 12 and the housing 16. The adjustment is effected by retracting the strap 14 into the cassette within the housing 16.

A remote output unit 22 is connected to the housing 16 via a cable 24. The remote output unit 22 plugs into the housing 16 via a releasable plug and socket connection, and can easily be removed. The remote output unit 22 includes red, yellow and green lamps.

The pressure sensors 18, 20 are each provided with a foam covering. The foam covering may be a self skinning foam. This creates an extended actuation area for the pressure sensors 18, 20, ensuring that contact with the pressure sensors 18, 20 will be correctly detected, as the user moves around in the seat.

In operation, the red lamp on the remote output unit 22 illuminates when no contact with either of pressure sensors 18, 20 is detected. The yellow lamp illuminates when contact with only one out of the pressure sensors 18, 20 is detected, and the green lamp illuminates when contact with both of the pressure sensors 18, 20 is detected. A vibrating actuator and audio transducer are included within the housing 16. The vibrating actuator and audio transducer provide a vibrating and/or audio alert when no contact is detected with at least one of the pressure sensors 18, 20.

A light sensor is provided on the housing 16. The light sensor provides a means for detecting whether a person is sat in the seat, and will turn the unit off if no person is detected, that is, when the light level on the light sensor is above a predetermined threshold.

If no contact is detected on either of the pressure sensors 18, 20, then the red lamp illuminates as described above. A timer is also started. If contact is not detected on at least one of the pressure sensors 18, 20 within a predetermined time period, for example, one minute, then the unit will switch off.

A USB interface is provided on the housing 16 connecting to the control unit. The USB interface allows readings from the pressure sensors 28, 20 to be fed to a personal computer or other device, for monitoring or recording posture, and/or for providing alternative output means, including on a personal computer display.

It is contemplated that, as an alternative to the retractable strap, elastic braces may be provided, extending from the housing 16. The braces could in use be stretched up the back of the chair, to ensure grip of the hook 12 on the top of the chair. It is also envisaged that some embodiments may include more than two pressure sensors. The sensors may also be adjustably positionable on the strap 14.

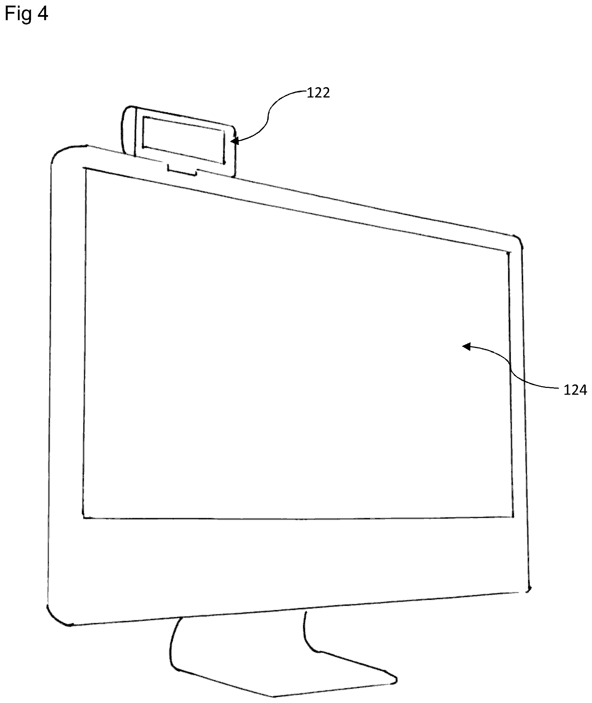

In an alternative embodiment, shown in Figure 4, a remote output unit 122 is provided as a separate display device or wand wirelessly connected to the control unit within the housing 16. The remote output unit 122 may be provided with a resilient clip or clamp means for enabling it to be attached to a computer screen 124, other electronic device or a fixture. Alternatively it may be placed on a desk or the like. Webcam may be built into the remote output unit 122, adding to its functionality.

The wand is adapted to display images of exercise routines at timed intervals, for example, at 40 minute intervals, to encourage mobility exercises being performed. It may also display a prompt to have a drink. The exercises can be uploaded from a computer and preferably directly from an internet site displayed on the web browser of the computer. In one embodiment, a one touch button is provided for facilitating display of the selected website on the browser. Preferred exercise routines can be downloaded and displayed, which may be changed, as desired by uploading new exercise routines.

The housing 16 may incorporate a rechargeable battery. Similarly, the remote output unit 122 may be powered by a rechargeable battery.

In use the device is highly beneficial and has the following benefits:
- Reinforces the delivery of display screen equipment health and safety
- Supports investment in ergonomic chairs
- Provides a long term approach to addressing back pain
- Helps to prevent repetitive strain injury
- Supports corporate health goals
- Helps to reduce sickness absence and associated costs at work

The device not only monitors a person's seated posture and advises when it should be corrected, but also provides suggested mobility exercises at timed intervals throughout the day or seated period. The exercises can be changed, as desired through a link with a website, where useful information relating to posture and mobility can also be displayed. Drinking prompts are also displayed at timed intervals to encourage good hydration.
It is understood that variations may be made in the foregoing without departing from the scope of the invention as claimed.

## Claims

1. A posture monitoring device (10) for use with a seat, comprising at least two pressure sensors (18, 20), a mount for securing the pressure sensors to a seat back (102), the mount including a hook (12) for fitting over the seat back and a flexible strap (14) depending from the hook, and output means (22, 122) for alerting when the pressure measured on at least one of the pressure sensors falls outside of a predetermined range,
**characterised in that**
the sensors are spaced apart along the length of the strap and mounted to the strap.

2. A posture monitoring device as claimed in claim 1, in which the hook is a malleable hook.

3. A posture monitoring device as claimed in claim 1 or 2, comprising a housing (16) and a controller provided in the housing.

4. A posture monitoring device as claimed in claim 3, in which the housing has a triangular cross section for positioning at the base of a seat back and is connected to the flexible strap.

5. A posture monitoring device as claimed in any preceding claim, in which the output means is further configured to indicate when the pressure on both of the pressure sensors falls outside of a predetermined range.

6. A posture monitoring device as claimed in any preceding claim, in which the output means includes a first lamp which illuminates when the pressure on both of the pressure sensors is within a predetermined range, a second lamp which illuminates when the pressure on at least one of the pressure sensors is outside of the predetermined range, and a third lamp which illuminates when the pressure on both of the pressure sensors is outside of the predetermined range.

7. A posture monitoring device as claimed in any preceding claim, configured to provide an audible alert when the pressure on both of the pressure sensors falls outside of a predetermined range.

8. A posture monitoring device as claimed in any preceding claim, configured to provide a vibrating alert when the pressure on both of the pressure sensors falls outside of a predetermined range.

9. A posture monitoring device as claimed in claim 8, when dependent on claim 3, in which the vibrating alert is provided by a transducer mounted in the housing.

10. A posture monitoring device as claimed in any preceding claim, configured to automatically switch off the device after a predetermined time period during which no pressure is detected by either sensor.

11. A posture monitoring device as claimed in any preceding claim, in which a light sensor is provided, the device being configured for automatically switching off when the light level detected by the sensor is greater than a predetermined threshold.

12. A posture monitoring device as claimed in any preceding claim, in which the output means is provided by a remote output unit (122).

13. A posture monitoring device as claimed in claim 12, when dependent on claim 3, in which the remote output unit is wirelessly connected to the controller.

14. A posture monitoring device as claimed in claim 12 or 13, in which a display screen is provided on the remote output unit.

15. A posture monitoring device as claimed in any one of claims 12 to 14, in which the remote output unit is adapted to connect to a computer, enabling downloading of selected mobility exercise images to the remote output unit for display.

## Patentansprüche

1. Gerät zur Überwachung der Körperhaltung (10) zur Verwendung mit einem Sitz, das mindestens zwei Drucksensoren (18, 20), eine Halterung zur Befestigung der Drucksensoren an einer Rückenlehne (102) umfasst, wobei die Halterung einen Haken (12) zur Befestigung über der Rückenlehne und einen vom Haken anhängenden flexiblen Riemen (14) und Ausgabemittel (22, 122) zum Warnen, wenn der gemessene Druck bei mindestens einem der Drucksensoren außerhalb eines vorbestimmten Bereichs fällt, umfasst, ***gekennzeichnet dadurch, dass*** die Sensoren im Abstand voneinander entlang der Länge des Riemens angeordnet und auf den Riemen montiert sind.

2. Gerät zur Überwachung der Körperhaltung gemäß Anspruch 1, in dem der Haken ein weicher Haken ist.

3. Gerät zur Überwachung der Körperhaltung gemäß Anspruch 1 oder 2, das ein Gehäuse (16) und eine Steuerung umfasst, die im Gehäuse bereitgestellt wird.

4. Gerät zur Überwachung der Körperhaltung gemäß Anspruch 3, in dem das Gehäuse über einen dreieckigen Querschnitt zur Positionierung am Untersatz der Rückenlehne verfügt und mit dem flexiblen Riemen verbunden ist.

5. Gerät zur Überwachung der Körperhaltung gemäß vorstehenden Ansprüchen, in dem das Ausgabenmittel weiter konfiguriert ist, um anzugeben, wenn der Druck an beiden Drucksensoren außerhalb eines vorbestimmten Bereichs fällt.

6. Gerät zur Überwachung der Körperhaltung gemäß vorstehenden Ansprüchen, in dem das Ausgabenmittel eine erste Lampe, die aufleuchtet, wenn der Druck an beiden Drucksensoren innerhalb eines vorbestimmten Bereichs liegt, eine zweite Lampe, die aufleuchtet, wenn der Druck an mindestens einem der Drucksensoren außerhalb des vorbestimmten Bereichs liegt, und eine dritte Lampe, die aufleuchtet, wenn der Druck an beiden Drucksensoren außerhalb des vorbestimmten Bereichs liegt, umfasst.

7. Gerät zur Überwachung der Körperhaltung gemäß vorstehenden Ansprüchen, das dafür konfiguriert ist, eine hörbare Warnung bereitzustellen, wenn der Druck an beiden Drucksensoren außerhalb eines vorbestimmten Bereichs fällt.

8. Gerät zur Überwachung der Körperhaltung gemäß vorstehenden Ansprüchen, das dafür konfiguriert ist, eine Vibrationswarnung bereitzustellen, wenn der Druck an beiden Drucksensoren außerhalb eines vorbestimmten Bereichs fällt.

9. Gerät zur Überwachung der Körperhaltung gemäß Anspruch 8, in dem die Vibrationswarnung, wenn abhängig von Anspruch 3, von einem in das Gehäuse montierten Wandler bereitgestellt wird.

10. Gerät zur Überwachung der Körperhaltung gemäß vorstehenden Ansprüchen, das dafür konfiguriert ist, das Gerät nach einem vorbestimmten Zeitraum, in dem von keinem der Sensoren Druck erkannt wird, automatisch auszuschalten.

11. Gerät zur Überwachung der Körperhaltung gemäß vorstehenden Ansprüchen, in dem ein Lichtsensor bereitgestellt wird, wobei das Gerät dafür konfiguriert ist, sich automatisch auszuschalten, wenn die vom Sensor erfasste Lichtstärke größer als ein vorbestimmter Schwellenwert ist.

12. Gerät zur Überwachung der Körperhaltung gemäß vorstehenden Ansprüchen, in dem das Ausgabenmittel durch ein Remote-Ausgabegerät (122) bereitgestellt wird.

13. Gerät zur Überwachung der Körperhaltung gemäß Anspruch 12, in dem das Remote-Ausgabegerät, wenn abhängig von Anspruch 3, drahtlos mit der Steuerung verbunden ist.

14. Gerät zur Überwachung der Körperhaltung gemäß Anspruch 12 oder 13, in dem ein Bildschirm auf dem Remote-Ausgabegerät bereitgestellt wird.

15. Gerät zur Überwachung der Körperhaltung gemäß Ansprüchen 12 bis 14, in dem das Remote-Ausgabegerät darauf angepasst ist, sich mit einem Computer zu verbinden, und das Herunterladen ausgewählter Bewegungsübungsbilder zur Anzeige auf das Remote-Ausgabegerät ermöglicht.

## Revendications

1. Dispositif de contrôle de la posture (10) conçu pour être utilisé avec un siège, et comprenant au moins deux capteurs de pression (18, 20), une monture pour fixer les capteurs de pression sur le dossier (102) du siège, la monture comprenant un crochet (12) pour le montage sur le dossier du siège et une courroie flexible (14) tributaire du crochet, et un avertisseur (22, 122) alertant l'utilisateur lorsque la pression mesurée sur au moins un des capteurs de pression se trouve hors de la plage de pressions prédéterminée, ***caractérisé en ce que*** les capteurs sont espacés sur la longueur de la courroie et montés sur cette dernière.

2. Dispositif de contrôle de la posture selon la revendication 1, dans lequel le crochet est un crochet malléable

3. Dispositif de contrôle de la posture selon la revendication 1 ou 2, comprenant un boîtier (16), et un régulateur placé dans le boîtier.

4. Dispositif de contrôle de la posture selon la revendication 3, le boîtier présentant une section transversale triangulaire permettant son positionnement à la base du dossier du siège, et étant raccordé à la courroie flexible.

5. Dispositif de contrôle de la posture selon une quelconque des revendications précédentes, dans lequel l'avertisseur est également configuré pour indiquer quand la pression sur les deux capteurs de pression se trouve hors de la plage de pressions prédéterminée.

6. Dispositif de contrôle de la posture selon une quelconque des revendications précédentes, dans lequel l'avertisseur comprend un premier voyant lumineux qui s'allume lorsque la pression sur les deux capteurs de pression est comprise dans la plage de pressions prédéterminée, et un deuxième voyant lumineux qui s'allume lorsque la pression sur au moins un des deux capteurs de pression se trouve hors de la plage de pressions prédéterminée, et un troisième voyant lumineux qui s'allume lorsque la pression sur les deux capteurs de pression se trouve hors de la plage de pressions prédéterminée.

7. Dispositif de contrôle de la posture selon une quelconque des revendications précédentes, configuré pour émettre une alarme sonore lorsque la pression sur les deux capteurs de pression se trouve hors de la plage de pressions prédéterminée.

8. Dispositif de contrôle de la posture selon une quelconque des revendications précédentes, configuré pour émettre une alarme vibratoire lorsque la pression sur les deux capteurs de pression se trouve hors de la plage de pressions prédéterminée

9. Dispositif de contrôle de la posture selon la revendication 8, lorsqu'il est tributaire de la revendication 3, dans lequel l'alarme vibratoire est déclenchée par un transducteur monté dans le boîtier.

10. Dispositif de contrôle de la posture selon une quelconque des revendications précédentes, configuré pour désactiver automatiquement le dispositif au bout d'une période prédéterminée au cours de laquelle aucun des capteurs n'a détecté une pression.

11. Dispositif de contrôle de la posture selon une quelconque des revendications précédentes, comprenant un capteur de lumière, le dispositif étant configuré pour se désactiver automatiquement quand le niveau lumineux détecté par le détecteur dépasse un seuil prédéterminé.

12. Dispositif de contrôle de la posture selon une quelconque des revendications précédentes, l'avertisseur étant assuré par une télécommande (122)

13. Dispositif de contrôle de la posture selon la revendication 12, lorsqu'il est tributaire de la revendication 3, la télécommande étant connectée sans fil au régulateur.

14. Dispositif de contrôle de la posture selon la revendication 12 ou 13, dans lequel la télécommande est dotée d'un écran d'affichage.

15. Dispositif de contrôle de la posture selon la revendication 12 à 14, dans lequel la télécommande est adaptée pour être branchée sur un ordinateur, afin de permettre le téléchargement, à la télécommande, d'images d'exercices de mobilité sélectionnés pour les afficher.
